# EUROPÄISCHE PATENTSCHRIFT

(11) **EP 2 161 597 B1**
(45) Veröffentlichungstag und Bekanntmachung des Hinweises auf die Patenterteilung: **04.06.2014**
(21) Anmeldenummer: 08163608.6
(22) Anmeldetag: 03.09.2008
(51) Int. Cl.: G02B 5/124, G02B 27/00, A61B 19/00

(54) **Bildgestütztes Operationssystem**
Image-assisted operation system
Système d'opération assisté par image

(43) Veröffentlichungstag der Anmeldung: 10.03.2010
(73) Patentinhaber: Brainlab AG, 85622 Feldkirchen (DE)
(72) Erfinder: Maier, Christian, 80802 München (DE)
(74) Vertreter: Schwabe - Sandmair - Marx

(56) Entgegenhaltungen:
- EP-A- 1 774 922
- EP-A1- 1 908 406
- WO-A1-99/58065
- FR-A- 2 691 129
- US-A- 3 977 765
- US-A- 5 589 981
- US-A1- 2006 241 388
- US-A1- 2007 035 835
- US-A1- 2008 131 115

## Beschreibung

Die vorliegende Erfindung betrifft ein bildgestütztes Operationssystem mit einem Retroreflektor.

Bei bildgestützten Operationssystemen werden Objekte, beispielsweise medizinische Instrumente oder Knochen, mit Markervorrichtung versehen, um die Lage, also die Position und/oder Ausrichtung, des Objekts ermitteln zu können. Bei den Markervorrichtungen handelt es sich üblicherweise um eine oder mehrere diffus reflektierende Kugeln, die das von einer Lichtquelle ausgesendete Licht reflektieren. Das reflektierte Licht wird von einem Sensor, beispielsweise einer 3D-Kamera, erfasst. In einer Recheneinheit wird der Mittelpunkt jeder Reflektion ermittelt und für die nachfolgende Lageberechnung als Mittelpunkt einer Kugel angesehen. Ein Problem tritt auf, wenn eine Kugel teilweise verdeckt wird und der Sensor daher die Reflektion nur teilweise erfassen kann. Auch in diesem Fall wird der Mittelpunkt der Reflektion als Mittelpunkt der Kugel angenommen, der jedoch nicht dem tatsächlichen Mittelpunkt der Kugel entspricht. Dies hat üblicherweise einen Fehler in der Berechnung der Lage des Objekts zur Folge.

Dies wird vermieden durch den Einsatz eines Retroreflektors in der Markervorrichtung. Ein Retroreflektor reflektiert einfallende Strahlung, beispielsweise Licht, parallel zur Einfallsrichtung zurück. Dies geschieht aufgrund mehrfacher Reflektion im Retroreflektor, wobei jeder einfallende Strahl eine Parallelverschiebung erfährt. Aufgrund dieser Parallelverschiebung ist der komplette Strahlengang unterbrochen, sobald der Retroreflektor zu großflächig verdeckt ist. Der Retroreflektor kann demnach nur dann vom Sensor erfasst werden, wenn er ausreichend sichtbar und damit korrekt lokalisierbar ist.

Bei einem bildgestützten Operationssystem ist es oftmals notwendig, dass der Retroreflektor rundum, also in alle Raumrichtungen, retroreflektierende Eigenschaften besitzt. Ein solcher Retroreflektor ist beispielsweise offenbart in der Offenlegungsschrift US 2008/0131115 A1. Der dort gezeigte Retroreflektor besteht aus acht Würfelecken, wobei die Spitzen der Würfelecken aneinander angrenzen und jede Würfelecke aus drei reflektierenden Flächen gebildet wird. Der Retroreflektor dient dazu, ein Schallfeld zu detektieren, indem die retroreflektierende Strahlung von einer Membran moduliert wird, die sich in einer der reflektierenden Flächen befindet.

Das Dokument US 2006/241388 A1 offenbart ein Lokalisationssystem mit einem Infrarot-Emitter, einem Retroreflektor und einem Infrarot-Detektor.

Das Dokument US 5,589,981 offenbart einen Retroreflektor mit acht Würfelecken, deren Spitzen aneinander angrenzen und der von einem Glaskubus umgeben ist.

Es ist die Aufgabe der vorliegenden Erfindung, ein bildgestütztes Operationssystem mit einem Retroreflektor bereitzustellen, das robust aufgebaut ist.

Diese Aufgabe wird gelöst durch ein bildgestütztes Operationssystem mit einem Retroreflektor gemäß Anspruch 1.

Der Retroreflektor des bildgestützten Operationssystems umfasst acht Würfelecken und umfasst insbesondere nur genau acht Würfelecken, wobei die Spitzen der Würfelecken aneinander angrenzen und jede Würfelecke aus drei reflektierenden Flächen gebildet wird. Die Spitzen der Würfelecken berühren sich demnach in einem Mittelpunkt des Retroreflektors. Der Mittelpunkt des Retroreflektors ist der Punkt, in dem sich die Ebenen, in denen die reflektierenden Flächen des Retroreflektors liegen, schneiden. Die Spitze einer Würfelecke ist derjenige Punkt, an dem die drei reflektierenden Flächen zusammentreffen.

Erfindungsgemäß weist der Retroreflektor einen Verschmutzungsschutz auf, der eine Ablagerung von Schmutz in den Würfelecken verhindert. Dadurch lässt sich der Retroreflektor nach einem Einsatz, beispielsweise einer Operation, einfach reinigen, insbesondere desinfizieren. Insbesondere ist der Verschmutzungsschutz ausgebildet, einen (haftenden) Kontakt von Schmutz, insbesondere Staub oder Flüssigkeit, auf den reflektierenden Flächen zu verhindern.

Es sei angemerkt, sich die Begriffe "reflektierend" und "transparent" im Rahmen dieses Dokuments auf Strahlung einer Wellenlänge beziehen, die von dem Retroreflektor reflektiert werden soll. Dabei handelt es sich beispielsweise um sichtbares Licht oder insbesondere Infrarotstrahlung.

Erfindungsgemäß umfasst der Verschmutzungsschutz eine Füllung der Würfelecken mit transparentem Material. Dadurch wird verhindert, dass sich Verschmutzungen in den Kanten und besonders der Spitze der Würfelecken ablagern. Dabei ist die Füllung so ausgestaltet, dass sie eine glatte Oberfläche des Retroreflektors erzeugt. Eine solche Oberfläche ist beispielsweise die einer Kugel oder eines Quaders, insbesondere eines Würfels.

Die reflektierenden Flächen der Würfelecken werden beispielsweise durch reflektierend beschichtete Wände gebildet. Eine Wand kann auch beidseitig reflektierend beschichtet sein und somit zwei reflektierende Flächen zweier benachbarter Würfelecken bilden. In einer anderen Ausgestaltungsform der Erfindung werden die reflektierenden Flächen durch Wände aus reflektierendem Material gebildet. Auch in diesem Fall kann eine Wand zwei reflektierende Flächen zweier benachbarter Würfelecken bilden.

Bei einer Füllung der Würfelecken mit transparentem Material besteht insbesondere die Möglichkeit, dass die reflektierenden Flächen der Würfelecken durch eine teilweise Beschichtung der Füllung gebildet werden. Die Füllung wird beispielsweise mit Hilfe einer Form gegossen, so dass eine Achtelkugel, eine gleichseitige Pyramide mit dreieckiger Grundfläche oder ein Quader entsteht. Anschließend werden die drei geraden Flächen der Achtelkugel, die drei Seitenflächen der Pyramide oder drei Seitenflächen des Quaders mit einer reflektierenden Beschichtung versehen und anschließend zusammengefügt, um den Retroreflektor zu bilden. Dabei berühren sich jeweils zwei beschichtete Flächen. Alternativ wird von den beiden Flächen, die sich beim Zusammenfügen berühren, nur eine mit einer reflektierenden Beschichtung versehen.

Bevorzugt weist der Retroreflektor einen Haltestab auf, mittels dessen der Retroreflektor an einem Objekt befestigt werden kann. In einer Ausgestaltungsform der Erfindung stimmt die Mittelachse des Haltestabs mit der Schnittlinie zweier reflektierender Flächen einer der Würfelecken überein. In diesem Fall verläuft oder endet der Haltestab in einer Kante einer Würfelecke oder mehrerer benachbarter Würfelecken. Der Haltestab bildet damit zumindest teilweise die Verlängerung einer Kante, die von zwei reflektierenden Flächen einer Würfelecke gebildet wird. Diese Anordnung hat den Vorteil, dass der Haltestab die Reflektionseigenschaften keine der Würfelecken beeinträchtigt.

In einer alternativen Ausgestaltungsform ist der Haltestab mittig in einer der Würfelecken angeordnet und fest mit der Spitze dieser Würfelecke verbunden. Eine mittige Anordnung bedeutet, dass ein Punkt auf der Mittelachse des Haltestabs von jeder der drei reflektierenden Flächen der Würfelecke den gleichen Abstand aufweist. Anders ausgedrückt bildet die Projektion der Mittelachse des Haltestabs in eine der reflektierenden Flächen die Winkelhalbierende zwischen den beiden anderen reflektierenden Flächen. Diese Anordnung behindert zwar die Retroreflektion in der entsprechenden Würfelecke, diese ist jedoch dem markierten Objekt zugewandt und somit bei der Lageerfassung ohnehin von diesem Objekt verdeckt.

Bei einem Herstellungsverfahren für einen Retroreflektor werden zunächst acht aus jeweils drei reflektierenden Flächen bestehende Würfelecken derart angeordnet, dass ihre Spitzen aneinander angrenzen. Anschließend werden die Würfelecken mit einem Verschmutzungsschutz versehen. Die reflektierenden Flächen werden beispielsweise aus einem Trägermaterial gebildet, das mit einer reflektierenden Schicht versehen ist, oder die reflektierenden Flächen werden aus Wänden gebildet, die aus reflektierendem Material bestehen.

Erfindungsgemäß wird der Verschmutzungsschutz dadurch erzeugt, dass die Würfelecken mit einem transparenten Material gefüllt werden. Dabei werden die Würfelecken beispielsweise ausgegossen. Durch die Gestaltung der Füllung lassen sich verschiedene Oberflächenformen des Retroreflektors erzeugen.

In einem alternativen Herstellungsverfahren werden acht Teilkörper aus transparentem Material erzeugt. Anschließend werden Seitenflächen der Teilkörper mit einem reflektierenden Material beschichtet und die beschichteten Teilkörper zu dem Retroreflektor zusammengesetzt. Eine Beschichtung kann durch jede geeignete Beschichtungstechnik erfolgen, beispielsweise durch Abscheiden, Besprühen oder Bekleben. Entweder werden alle drei Seitenflächen jedes Teilkörpers beschichtet, die im zusammengesetzten Retroreflektor eine Seitenfläche eines anderen Teilkörpers berühren, oder von zwei sich berührenden Seitenflächen benachbarter Teilkörper wird nur eine beschichtet.

Bevorzugt werden die acht Teilkörper durch drei Schnitte aus einem transparenten Vollkörper erzeugt, der Vollkörper wird also geachtelt. Dabei sind die drei Schnitte bevorzugt zueinander senkrecht. Geht beim Durchführen der Schnitte Material verloren, beispielsweise bei einem spanenden Verfahren, werden die Schichten bevorzugt so dick ausgebildet, dass sie das entfernte Material des Vollkörpers kompensieren. Der Vollkörper hat demnach nach dem Zusammensetzen wieder exakt seine Ursprungsform.

Die Erfindung betrifft ein bildgestütztes Operationssystem mit einer Lichtquelle, mindestens einem vorstehend beschriebenen Retroreflektor zur Reflektion des Lichts der Lichtquelle, mindestens einen Detektor zur Erfassung des reflektierten Lichts und eine Recheneinheit zur Berechnung der Position des Retroreflektors aus dem Ausgangssignal des Detektors. Bei dem Detektor handelt es sich beispielsweise um eine Kamera, insbesondere eine 3D-Kamera. Die Lichtquelle befindet sich bevorzugt in unmittelbarer Nähe des Detektors. Aus der Lage des Retroreflektors ergibt sich die Lage des mit dem Retroreflektor markierten Objekts.

Weiterhin betrifft die Erfindung die Verwendung eines Retroreflektors aus acht Würfelecken, wobei die Spitzen der Würfelecken aneinander angrenzen und jede Würfelecke aus drei reflektierenden Flächen gebildet wird, in einem bildgestützten Operationssystem.

Die vorliegende Erfindung soll anhand eines Ausführungsbeispiels näher erläutert werden. Dabei zeigt:
- Figur 1: eine Würfelecke,
- Figur 2: eine erste Anordnung aus acht Würfelecken,
- Figur 3: die Anordnung aus Figur 2 umgeben von einer Schale,
- Figur 4: eine zweite Anordnung aus acht Würfelecken,
- Figur 5: eine Schnittdarstellung eines Retroreflektors,
- Figur 6: ein bildgesteuertes Operationssystem und
- Figur 7: eine Frontansicht einer 3D-Kamera.

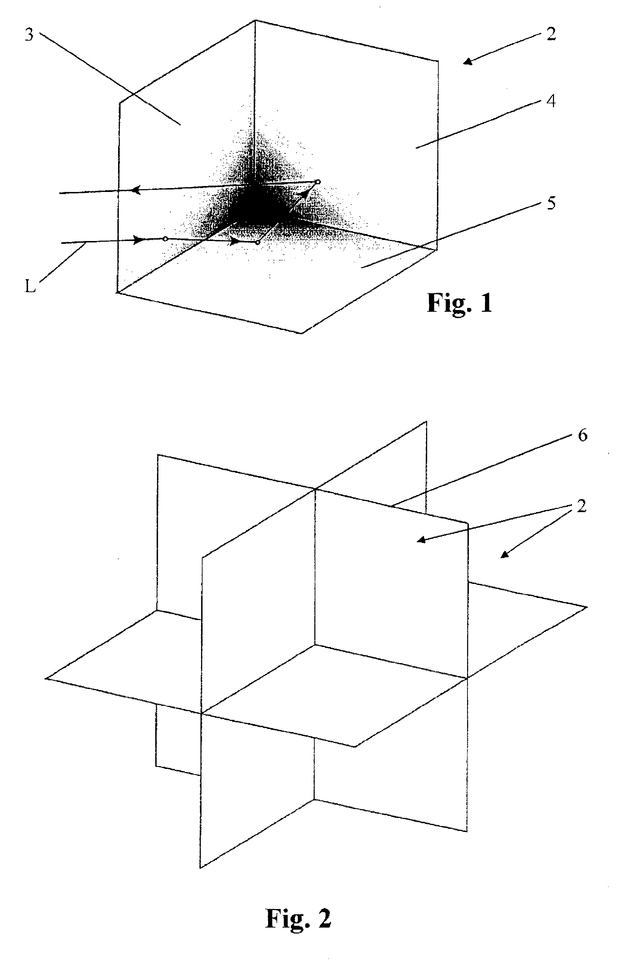

Dargestellt in der Figur 1 ist schematisch eine einzelne Würfelecke 2, die auch als Dreifachspiegel bezeichnet wird. Die Würfelecke 2 besteht aus drei jeweils zueinander senkrechten Flächen 3, 4 und 5. Die Flächen 3, 4 und 5 bestehen aus Wänden, die mit einer reflektierenden Beschichtung versehen sind, beispielsweise einer Beschichtung aus Silber, Aluminium, Kupfer, Gold oder Mischungen dieser Metalle. Die reflektierende Beschichtung kann mit einer schmutzabweisenden Nano-Beschichtung versehen sein. Der beispielhafte, auf die Würfelecke 2 treffende Lichtstrahl L wird zunächst an der Fläche 3, dann an der Fläche 5 und zuletzt an der Fläche 4 reflektiert, sodass er von der Würfelecke 2 insgesamt mit einem Versatz parallel zur Einfallsrichtung zurückreflektiert wird.

Dargestellt in der Figur 2 ist eine erste Anordnung aus acht Würfelecken 2, deren Spitzen im Mittelpunkt der Anordnung aneinander angrenzen. Die reflektierenden Flächen der Würfelecken 2 werden von reflektierend beschichteten Wänden gebildet, wobei jede Wand beidseitig beschichtet ist und somit zwei reflektierende Flächen für zwei benachbarte Würfelecken 2 bildet. Die reflektierenden Flächen und damit die Wände einer jeden Würfelecke 2 stehen jeweils senkrecht aufeinander, so dass jeweils vier der insgesamt zwölf Wände in einer gemeinsamen Ebene liegen. Mit der in der Figur 2 gezeigten Anordnung kann ein aus einer beliebigen Raumrichtung einfallender Lichtstrahl parallel zu seiner Einfallsrichtung zurückreflektiert werden.

Die reflektierenden Flächen haben jeweils die Form eines Rechtecks, insbesondere eines Quadrats, sodass auch die Kanten 6 der Anordnung aus acht Würfelecken 2 jeweils die Form eines Rechtecks bzw. eines Quadrates aufweisen. Die Kanten 6 der Anordnung setzen sich aus denjenigen Kanten der reflektierenden Flächen zusammen, die nicht an andere reflektierende Flächen angrenzen. Jede der Kanten 6 besteht aus den Kanten derjenigen reflektierenden Flächen, die in der gleichen Ebene liegen.

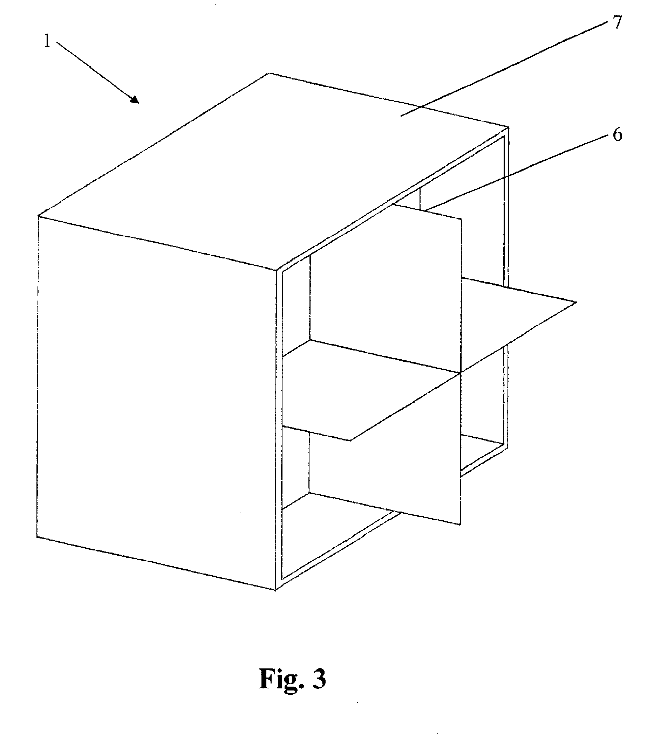

Die Figur 3 zeigt einen nicht erfindungsgemäßen Retroreflektor 1, bei dem die Anordnung aus acht Würfelecken 2 aus der Figur 2 mit einer transparenten Schale 7 in Form eines hohlen Quaders, beispielsweise eines hohlen Würfels, umgeben wurde. Die Schale 7 ist in der Figur 3 geschnitten dargestellt. Dabei ist die Größe des Quaders bzw. Würfels so gewählt, dass die Kanten 6 der Wände der Würfelecken 2 an der Innenfläche des Quaders bzw. Würfels anliegen. Bevorzugt sind die Kanten 6 fest mit der Innenfläche der Schale 7 verbunden, beispielsweise verklebt, wodurch sich die Stabilität der Würfelecken 2 erhöht.

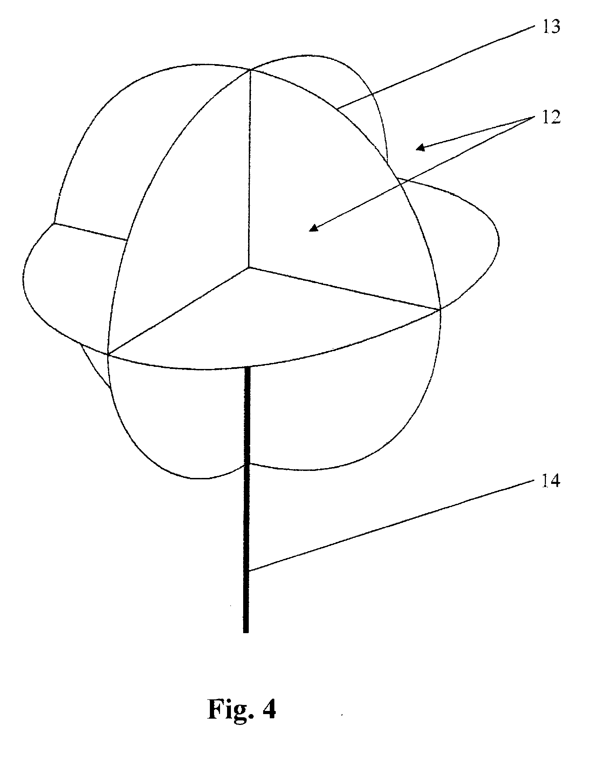

Die Figur 4 zeigt eine zweite Anordnung aus acht Würfelecken 12, wobei die reflektierenden Flächen der Würfelecken 12 keine Rechtecke sind, sondern Kreissektoren. Jeder Sektor entspricht einer viertel Kreisscheibe. Die reflektierenden Flächen sind jeweils an ihren radialen Kanten miteinander verbunden und stehen senkrecht aufeinander. Somit ergibt sich eine Anordnung von acht Würfelecken 12, wobei die drei Kanten 13 der Anordnung die Form von Vollkreisen aufweisen. Die Kanten 13 der Anordnung setzen sich aus denjenigen Kanten der reflektierenden Flächen zusammen, die nicht an andere reflektierende Flächen angrenzen. Jede der Kanten 13 besteht aus den Kanten derjenigen reflektierenden Flächen, die in der gleichen Ebene liegen. Die Anordnung aus acht Würfelecken bildet den Kern eines Retroreflektors 11.

Weiterhin ist ein Haltestab 14 vorgesehen, der sich vom Mittelpunkt der Anordnung aus den acht Würfelecken 12 aus entlang der Schnittlinie zweier Seitenflächen einer der Würfelecken 12 erstreckt. Der Haltestab 14 liegt somit genau in der Kante, an der sich zwei Wände einer Würfelecke 12 berühren. Dadurch werden die Reflektionseigenschaften dieser Würfelecke nicht beeinträchtigt. Der Haltestab 14 dient der Befestigung des Retroreflektors 11 an einem Objekt, dessen Lage zu bestimmen ist.

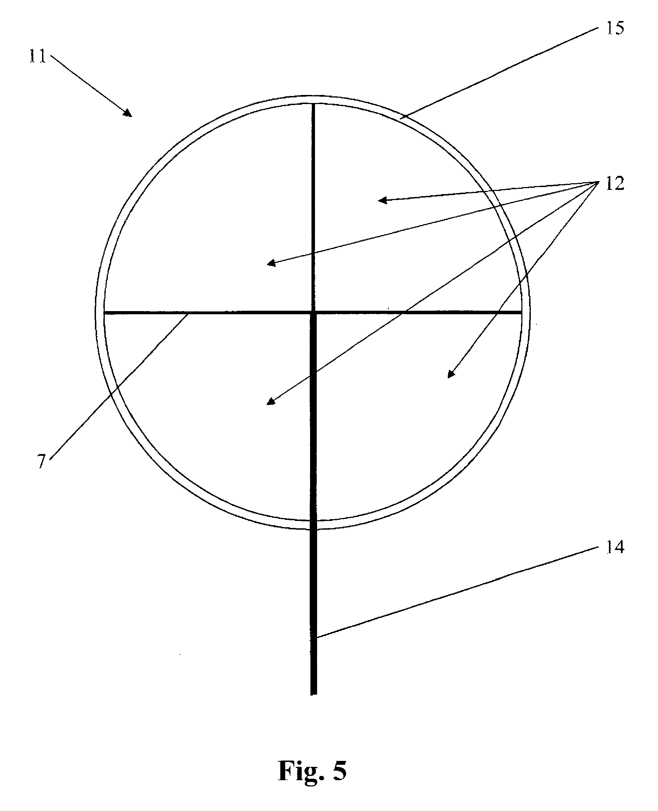

Die Figur 5 zeigt den Retroreflektor 11 in einer teilweise geschnittenen Seitenansicht, wobei die Anordnung aus acht Kugelecken 12 aus Figur 4 nicht erfindungsgemäß von einer Kugelschale 15 umhüllt wird. Die Kugelschale 15 besteht aus zwei Halbschalen. Die Anordnung aus acht Würfelecken 12 wird bei der Herstellung des Retroreflektors 11 in die erste Halbschale eingesetzt, danach wird die zweite Halbschale aufgesetzt und mit der ersten Halbschale verbunden, beispielsweise durch Verkleben. Dabei liegt die Verbindungslinie der beiden Halbschalen bevorzugt unmittelbar an einer der drei kreisförmigen Kanten 13 an. Dadurch bildet die Verbindungsstelle zwischen den beiden Halbschalen keine Störstelle im Strahlengang.

Der Außendurchmesser des Retroreflektors 11 liegt bevorzugt zwischen 1cm und 2cm inklusive der Intervallränder. Die Wandstärke der Wände der Würfelecken 12 sowie der Kugelschale 15 ist bevorzugt kleiner als ein Zwanzigstel des Durchmessers des Retroreflektors 11.

Alternativ zu einer Kapselung der Anordnungen aus acht Würfelecken 2 beziehungsweise 12 in einem hohlen Quader bzw. Würfel 7 oder einer Kugelschale 15 werden die Würfelecken 2, 12 erfindungsgemäß mit einem transparenten Material aufgefüllt. Im Falle der Anordnung aus Figur 2 wird die Füllung so ausgebildet, dass die äußere Oberfläche des Retroreflektors 1 die Form eines Quaders bzw. Würfels aufweist. Im Fall der Anordnung der Würfelecken in Figur 4 wird die Füllung so ausgestaltet, dass der Retroreflektor 11 die äußere Form einer Kugel aufweist.

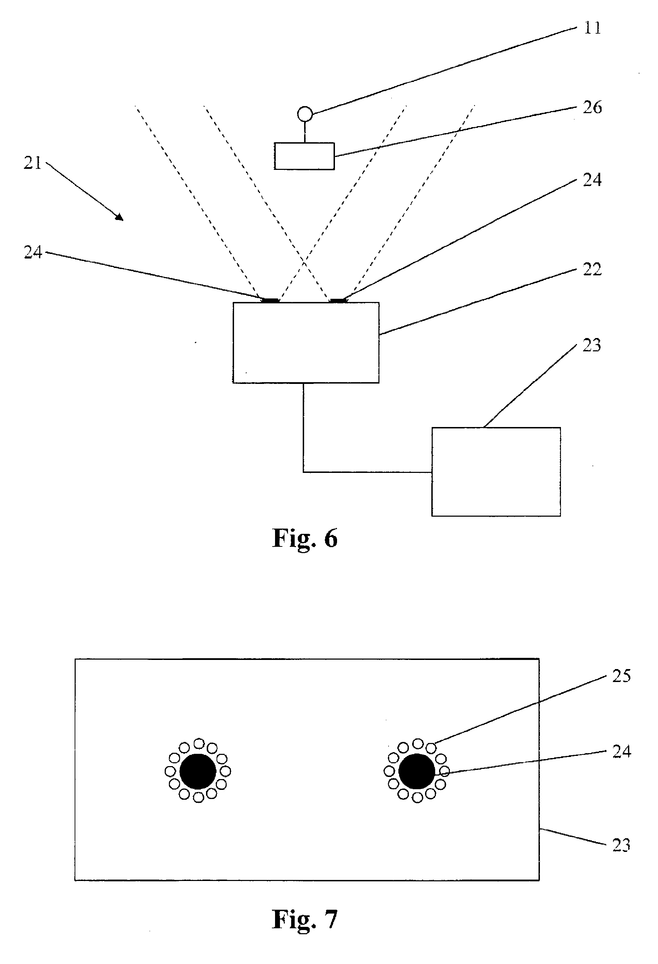

Die Figur 6 zeigt schematisch ein bildgestütztes Operationssystem 21 mit einer 3D-Kamera 22 und einer Recheneinheit 23. Die 3D-Kamera weist zwei Bildsensoren 24 auf, die jeweils ein zweidimensionales Bild aufnehmen. Die Erfassungsbereiche der Bildsensoren 24 sind durch gestrichelte Linien angedeutet.

Das bildgestützte Operationssystem 21 ist dazu eingerichtet, die Lage eines Objekts 26 zu bestimmen. Dazu ist das Objekt 26 fest mit einem Retroreflektor 11 verbunden. Die feste Verbindung zwischen Objekt 26 und Retroreflektor 11 hat zur Folge, dass deren Lagen zueinander eine feste Beziehung haben. Ist die Lage des Retroreflektors 11 bekannt, so kann daraus unmittelbar die Lage des Objekts 26 bestimmt werden.

Wie aus der schematischen Frontansicht der 3D-Kamera in Figur 7 erkennbar ist, ist jeder der Bildsensoren 24 von einem Ring aus Leuchtdioden 25 umgeben, wobei die Leuchtdioden 25 bevorzugt Licht im Infrarotbereich emittieren. Das emittierte Licht trifft auf den Retroreflektor 11 und wird auf die beiden Bildsensoren 24 zurückreflektiert. Die Ausgangssignale der Bildsensoren 24 werden an die Recheneinheit 23 übertragen und dort ausgewertet. Da die beiden Bildsensoren 24 den Retroreflektor 11 aus unterschiedlichen Positionen erfassen, kann die Position des Retroreflektors 11 und damit die Position des Objekts im Raum bestimmt werden. Sind an dem Objekt 26 mehrere Retroreflektoren 11 angeordnet, so kann aus den erfassten Positionen der Retroreflektoren 11 neben der Position auch die Ausrichtung des Objekts 26 bestimmt werden. Bevorzugt sind mindestens drei Retroreflektoren 11 zu einer Markervorrichtung zusammengefasst und mit dem Objekt 26 verbunden.

Im Rahmen der Patentansprüche ist es möglich, einzelne Merkmale der verschiedenen Ausführungsbeispiele miteinander zu kombinieren.

## Patentansprüche

1. Bildgestütztes Operationssystem (21), aufweisend eine Lichtquelle (25), mindestens einen Retroreflektor (1, 11) zur Reflektion des Lichts (L) der Lichtquelle (25), mindestens einen Detektor (24) zur Erfassung des reflektierten Lichts und eine Recheneinheit (23) zur Berechnung der Position des Retroreflektors (1, 11) aus dem Ausgangssignal des Detektors, **dadurch gekennzeichnet, dass** der Retroreflektor (1, 11) acht Würfelecken (2, 12) umfasst, wobei die Spitzen der Würfelecken (2, 12) aneinander angrenzen, jede Würfelecke (2, 12) aus drei reflektierenden Flächen (3, 4, 5) gebildet wird und ein Verschmutzungsschutz (7, 15) eine Ablagerung von Schmutz in den Würfelecken (2, 12) verhindert, wobei der Verschmutzungsschutz eine Füllung der Würfelecken (2, 12) mit transparentem Material umfasst, die so ausgestaltet ist, dass sie eine äußere Oberfläche des Retroreflektors (1) in Form eines Quaders, eines Würfels oder einer Kugel erzeugt.

2. Operationssystem (21) nach Anspruch 1, **dadurch gekennzeichnet, dass** der Verschmutzungsschutz eine transparente Schale (7, 15) umfasst, die die Würfelecken (2, 12) umgibt.

3. Operationssystem (21) nach Anspruch 1 oder 2, **dadurch gekennzeichnet, dass** die reflektierenden Flächen (3, 4, 5) durch eine teilweise Beschichtung der Füllungen gebildet werden.

4. Operationssystem (21) nach einem der Ansprüche 1 oder 2, **dadurch gekennzeichnet, dass** die reflektierenden Flächen (3, 4, 5) durch beschichtete Wände oder durch Wände aus reflektierendem Material gebildet werden.

5. Operationssystem (21) nach einem der Ansprüche 1 bis 4, **gekennzeichnet durch** einen Haltestab (14) am Retroreflektor (1, 11), dessen Mittelachse mit der Schnittlinie zweier reflektierender Flächen einer der Würfelecken (2, 12) übereinstimmt.

6. Operationssystem (21) nach einem der Ansprüche 1 bis 4, **gekennzeichnet durch** einen Haltestab (14) am Retroreflektor (1, 11), der mittig in einer der Würfelecken (12) angeordnet ist und fest mit der Spitze dieser Würfelecke (12) verbunden ist.

## Claims

1. An image-guided operation system (21) comprising a light source (25), at least one retro-reflector (1, 11) for reflecting the light (L) of the light source (25), at least one detector (24) for detecting the reflected light and a computational unit (23) for calculating the position of the retro-reflector (1, 11) from the output signal of the detector, **characterized in that** the retro-reflector (1, 11) comprises eight cube corners (2, 12), wherein the tips of the cube corners (2, 12) are adjacent to each other, each cube corner (2, 12) is formed from three reflective faces (3, 4, 5) and a contamination protector (7, 15) prevents contaminants from being deposited in the cube corners (2, 12), wherein the contaminant protector comprises a filling of the cube corners (2, 12) with transparent material, which is provided to constitute an outer surface of the retro-reflector (1) in the shape of a cuboid, a cube or a sphere.

2. The operation system (21) according to claim 1, **characterized in that** the contaminant protector comprises a transparent shell (7, 15) which surrounds the cube corners (2, 12).

3. The operation system (21) according to claim 1 or claim 2, **characterized in that** the reflective faces (3, 4, 5) are formed by partially coating the fillings.

4. The operation system (21) according to any one of claims 1 or 2, **characterized in that** the reflective faces (3, 4, 5) are constituted by coated walls or walls made of reflective material.

5. The operation system (21) according to any one of claims 1 to 4, **characterized by** a supporting rod (14) at the retro-reflector (1, 11), the center axis of which matches the line of intersection between two reflective faces of one of the cube corners (2, 12).

6. The operation system (21) according to any one of claims 1 to 4, **characterized by** a supporting rod (14) at the retro-reflector (1, 11), which is arranged centrally in one of the cube corners (12) and fixedly connected to the tip of this cube corner (12).

## Revendications

1. Système d'opération guidée par l'image (21) comportant une source lumineuse (25), au moins un rétro-réflecteur (1, 11) pour réfléchir la lumière (L) de la source lumineuse (25), au moins un détecteur (24) pour capter la lumière réfléchie et une unité de calcul (23) pour calculer la position du rétro-réflecteur (1, 11) à partir du signal de sortie du détecteur, **caractérisé en ce que** le rétro-réflecteur (1, 11) comprends huit coins de cube (2, 12), les sommets des coins de cube (2, 12) étant adjacents, chaque coin de cube (2, 12) étant formé par trois surfaces réfléchissantes (3, 4, 5) et qu'une protection anti-salissures (7, 15) empêche un dépôt de saleté dans les coins de cube (2, 12), la protection anti-salissures comprenant un remplissage des coins de cube (2, 12) avec un matériau transparent, configuré de telle manière qu'il forme une surface extérieure du rétro-réflecteur (1) en forme de pavé droit, de cube ou de boule.

2. Système d'opération (21) selon la revendication 1, **caractérisé en ce que** la protection anti-salissures comprends une coque transparente (7, 15) qui entoure les coins de cube (2, 12).

3. Système d'opération (21) selon la revendication 1 ou 2, **caractérisé en ce que** les surfaces réfléchissantes (3, 4, 5) sont formées par un revêtement partiel des remplissages.

4. Système d'opération (21) selon l'une des revendications 1 ou 2, **caractérisé en ce que** les surfaces réfléchissantes (3, 4, 5) sont formées par des parois enduites ou par des parois composées d'un matériau réfléchissant.

5. Système d'opération (21) selon l'une des revendications 1 à 4, **caractérisé par** une barre de maintien (14) sur le rétro-réflecteur (1, 11) dont l'axe central coïncide avec l'intersection de deux surfaces réfléchissantes de l'un des coins de cube (2, 12)

6. Système d'opération (21) selon l'une des revendications 1 à 4, **caractérisé par** une barre de maintien (14) sur le rétro-réflecteur (1, 11) qui est disposée de manière centrale dans l'un des coins de cube (12) et est solidaire du sommet de ce coin de cube (12).
